# EUROPEAN PATENT APPLICATION

(11) **EP 4 410 176 A1**
(43) Date of publication of application: **07.08.2024**
(21) Application number: 22875825.6
(22) Date of filing: 14.09.2022
(51) Int. Cl.: A61B 1/00, A61B 1/04, H04N 23/00, H04N 7/18, G02B 23/26

(54) **OPTICAL TRANSMITTER MODULE AND ENDOSCOPE**

(30) Priority: 01.10.2021 JP 2021162588
(71) Applicant: HOYA CORPORATION, Shinjuku-ku Tokyo 160-8347 (JP)
(72) Inventor: KOMATSU Masahiro, Tokyo 160-8347 (JP); TANI Nobuhiro, Tokyo 160-8347 (JP)
(74) Representative: Schaumburg und Partner Patentanwälte mbB
(86) International application number: PCT/JP2022/034298
(87) International publication number: WO 2023/053954

(57) **Abstract**

Provided are an optical transmitter module (30) and an endoscope (11) that can be more easily downsized with a more stable configuration.

The optical transmitter module (30) includes an image sensor (31), a laser element (32), a laser driver circuit (33), an optical fiber (34), heat dissipation wiring (35), electric signal wiring (36), and a layered substrate (37). The image sensor (31), the laser element (32), the laser driver circuit (33), at least a part of the optical fiber (34), at least a part of the heat dissipation wiring (35), at least a part of the electric signal wiring (36), and the layered substrate (37) are fixed to each other with a molding agent (61). The endoscope (11) includes the optical transmitter module (30) described above.

## Description

### Technical Field

The present invention relates to an optical transmitter module and an endoscope.

### Background Art

An endoscope in which an image sensor is built in a distal end of an insertion portion is known. In order to enable clear image observation, an image sensor having a large number of pixels is used. In a case where an image sensor having a large number of pixels is used in the endoscope, in order to transmit a signal between the image sensor and a signal processing device at a high speed, an optical transmitter module is incorporated in the endoscope, and an optical fiber is used for signal transmission.

Examples of an optical transmitter module using an optical fiber are described in Patent Literature 1 and Patent Literature 2.

### Citation List

### Patent Literature

Patent Literature 1: JP 6300442 B2
Patent Literature 2: WO 2018/092233 A

### Summary of Invention

### Technical Problem

However, there is a difficulty in downsizing a conventional optical transmitter module with a stable configuration.

For example, although an FPC substrate is used in Patent Literature 1, if the FPC substrate is configured to be orthogonal with an adhesive as described in Patent Literature 1, the configuration may be unstable at the time of manufacturing. In addition, since an extra space is formed in the optical transmitter module, downsizing is difficult.

The present invention has been made to solve such a problem, and aims to provide an optical transmitter module and an endoscope that can be more easily downsized with a more stable configuration.

### Solution to Problem

An optical transmitter module according to the present invention includes:
an image sensor, a laser element, a laser driver circuit, an optical fiber, a heat dissipation wiring, an electric signal wiring, and a layered substrate, wherein
the image sensor, the laser element, the laser driver circuit, at least a part of the optical fiber, at least a part of the heat dissipation wiring, at least a part of the electric signal wiring, and the layered substrate are fixed to each other with a molding agent.

An endoscope according to the present invention includes the optical transmitter module described above.

The present description is based on and claims priority pursuant to Japanese Patent Application No. 2021-162588, the entire disclosure of which is hereby incorporated by reference herein.

### Advantageous Effects of Invention

The optical transmitter module and the endoscope according to the present invention can be more easily downsized with a more stable configuration.

### Brief Description of Drawings

Fig. 1 is a diagram illustrating a configuration example of an endoscope system according to a first embodiment.
Fig. 2 is a diagram illustrating a configuration example of a distal end of the endoscope of Fig. 1.
Fig. 3 is a diagram illustrating a configuration example of an optical transmitter module of Fig. 2.
Fig. 4 is another diagram illustrating a configuration example of the optical transmitter module of Fig. 2.
Fig. 5 is a diagram illustrating a configuration example of a first cap member of Figs. 3 and 4.
Fig. 6 is a diagram illustrating an example of sealing with a molding agent.
Fig. 7 is a diagram illustrating a configuration example of an optical transmitter module according to a second embodiment.
Fig. 8 is a diagram illustrating a configuration example of an optical transmitter module according to a third embodiment.
Fig. 9 is a diagram illustrating a configuration example of an optical transmitter module according to a

### fourth embodiment.

### Description of Embodiments

Hereinafter, embodiments of the present invention will be described with reference to the accompanying drawings.

### First Embodiment

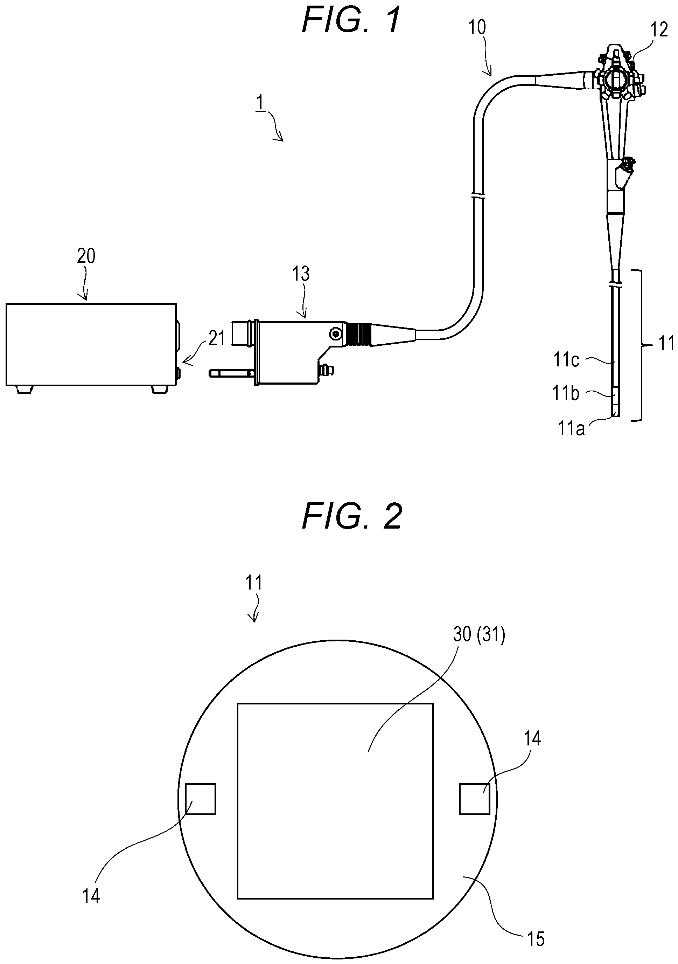

Fig. 1 is a diagram illustrating a configuration example of an endoscope system 1 according to the present embodiment. The endoscope system 1 may be what is called an electronic endoscope. As illustrated in Fig. 1, the endoscope system 1 is, for example, a dedicated system for medical use and includes an endoscope unit 10 and a processor 20.

As illustrated in Fig. 1, the endoscope unit 10 includes an endoscope 11 and an operation unit 12. The endoscope 11 includes, in order from the distal end, a hard portion 11a that is not deformed, a bend portion 11b that can actively bend in response to an operation, and a flexible portion 11c that can be passively deformed. A user of the endoscope system 1 operates the operation unit 12 to control a movement of the endoscope 11. For example, the bend portion 11b is bent depending on an operation of the operation unit 12. The bend portion 11b can be realized by using a known mechanism incorporated in a typical electronic scope, and is configured to bend the bend portion 11b by, for example, pulling an operation wire in conjunction with rotational operation of a knob included in the operation unit 12.

The endoscope 11 can insert a portion including the hard portion 11a into any body cavity in a living body, for example, into a bronchus, a biliary tract, a pancreas, a hepatic duct region, a urinary organ region, or the like.

The processor 20 integrally includes: a signal processing device that processes an image signal from the endoscope unit 10; and a light source device that applies light via the endoscope unit 10 to a body cavity to which no natural light can reach. Note that, in another embodiment, the signal processing device and the light source device may be configured separately.

A proximal end of the endoscope unit 10 is provided with a connector unit 13, and the processor 20 is provided with a connector unit 21. The connector unit 13 and the connector unit 21 each have a coupling structure corresponding to each other. Coupling of these structures electrically and optically connects the endoscope unit 10 and the processor 20.

The processor 20 functions as a controller that controls the entire endoscope system 1 and includes, for example, a computer provided with an arithmetic unit and a storage unit. Other functions and configurations of the endoscope unit 10 and the processor 20 (for example, the function of acquiring an image inside a body cavity or the like) can be appropriately designed by those skilled in the art based on known techniques. For example, the processor 20 performs various calculations on the basis of specific information of the endoscope unit 10 and generates a control signal. Furthermore, the processor 20 uses the generated control signal to control operations and timings of various circuits in the processor 20, thereby causing the endoscope unit 10 to move appropriately.

Note that, assuming that the endoscope unit 10 is connected to an appropriate processor other than the processor 20, the endoscope unit 10 may be provided independently.

Fig. 2 illustrates a configuration example of a distal end of the endoscope 11. Fig. 2 is a view (top view) of the distal end of the endoscope 11 as viewed from the distal end side in the axial direction. Note that, in the present description, the "axial direction" and the "radial direction" mean the axial direction and the radial direction of the endoscope 11, respectively. The endoscope 11 includes an optical transmitter module 30.

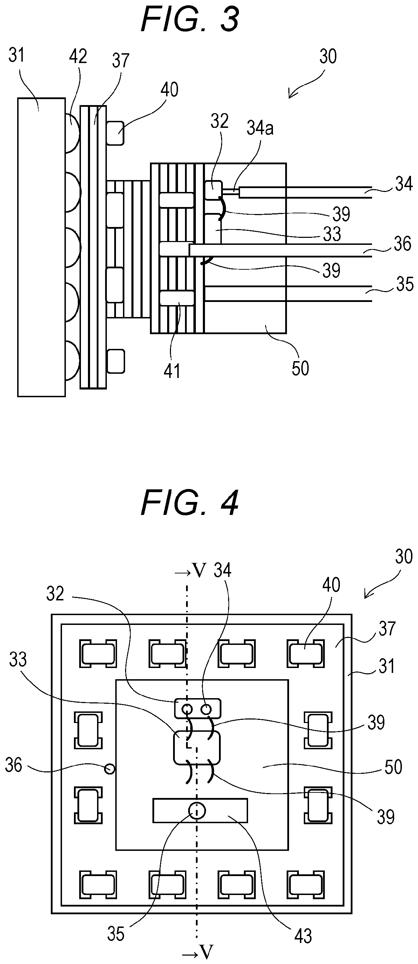

The configuration of the optical transmitter module 30 will be described later with reference to Figs. 3 and 4, for example. In Fig. 2, however, only an image sensor 31 of the optical transmitter module 30 appears. The optical transmitter module 30 is fixed in the endoscope 11 using a fixing member (for example, a rod 15). The structure of the fixing member is not limited to that shown in Fig. 2, and those skilled in the art can appropriately design the fixing member.

The endoscope 11 includes a light irradiation means 14. The light irradiation means 14 is provided at or near the distal end of the endoscope 11. The light irradiation means 14 is, for example, a ride guide, and more specifically, can be configured using an optical fiber.

Although not illustrated in Fig. 2, the endoscope 11 may further include other configurations included in a known endoscope. For example, the endoscope 11 may include a forceps channel, an air pipe, a water pipe, a water jet pipe, and the like. In that case, the distal end of the endoscope 11 illustrated in Fig. 2 may be provided with an opening or the like corresponding thereto.

Fig. 3 and Fig. 4 illustrate a configuration example of the optical transmitter module 30. Fig. 3 is a view (side view) of the optical transmitter module 30 as viewed from the radial direction, and Fig. 4 is a view (bottom view) of the optical transmitter module 30 as viewed from the rear end side in the axial direction.

The optical transmitter module 30 includes the image sensor 31 described above, a laser element 32, a laser driver circuit 33, at least one optical fiber 34 (two in the present embodiment), a heat dissipation wiring 35, at least one electric signal wiring 36, and a layered substrate 37. The image sensor 31, the laser element 32, the laser driver circuit 33, the optical fiber 34, the heat dissipation wiring 35, and the electric signal wiring 36 are mounted on the layered substrate 37.

The image sensor 31 is connected to the layered substrate 37 via a solder 42. The laser element 32 and the laser driver circuit 33 are mounted on the layered substrate 37 by die bonding and wire bonding, and in particular connected to the layered substrate 37 via wires 39. The laser element 32 and the laser driver circuit 33 constitute an electrical-optical converter that converts an electric signal output from the image sensor 31 into an optical signal.

The optical fiber 34 is connected to the laser element 32 and conveys the laser output from the laser element 32. The heat dissipation wiring 35 is connected to a ground potential pad 43 and discharges heat of the optical transmitter module 30 (for example, heat generated by the image sensor 31) via the ground potential pad 43.

A cable wiring pad 41 is provided on a side surface of the layered substrate 37 (that is, a surface parallel to the axis of the endoscope 11). Although three cable wiring pads 41 are illustrated in the example of Fig. 3, the number of the cable wiring pads 41 may be one or more. The electric signal wiring 36 is connected to the layered substrate 37 via the cable wiring pad 41. For convenience of illustration, only one electric signal wiring 36 is illustrated in the examples of Figs. 3 and 4, but the number of electric signal wiring 36 may be two or more, and for example, each of the cable wiring pads 41 may be connected to separated one electric signal wiring 36.

A passive component 40 (for example, a capacitor, a resistor, and the like) may be connected to the layered substrate 37. The passive component 40 may be connected to the electric signal wiring 36 via the layered substrate 37.

As described above, each component can be compactly arranged by a mounting style using the layered substrate 37, and the entire optical transmitter module 30 can be downsized.

The optical transmitter module 30 includes a first cap member 50 made of glass. As illustrated in Figs. 3 and 4, the first cap member 50 is disposed on a rear end side surface of the layered substrate 37 and covers the laser element 32, the laser driver circuit 33, the ground potential pad 43, and the like.

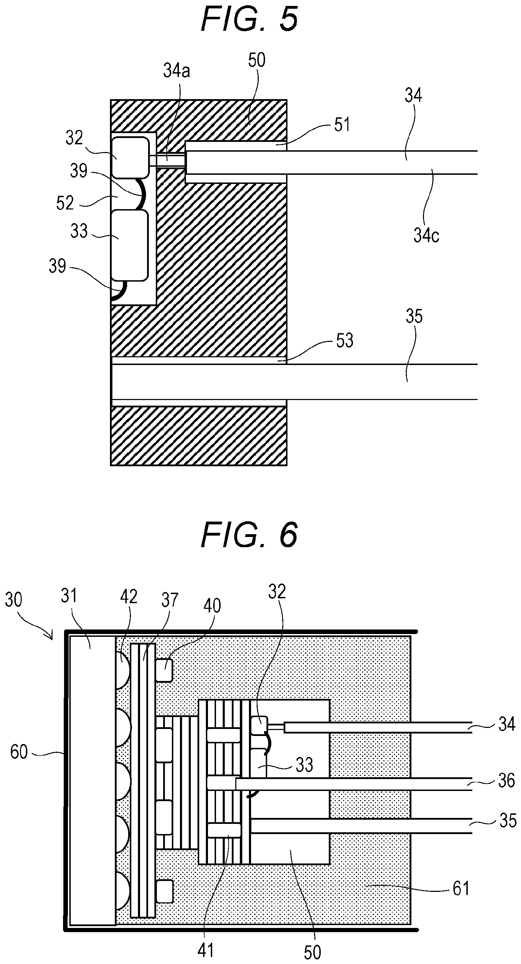

A configuration example of the first cap member 50 will be described with reference to Fig. 5. Fig. 5 is a displacement cross-sectional view of the first cap member 50 taken along line V-V in Fig. 4. The first cap member 50 includes a first through hole 51, a recess 52, and a second through hole 53.

The first through hole 51 penetrates the first cap member 50 in the axial direction and is connected to the recess 52. The first through hole 51 accommodates at least a part of the optical fiber 34. As in the present embodiment, in a case where the optical transmitter module 30 includes a plurality of the optical fibers 34, a single first through hole 51 may accommodate the plurality of optical fibers 34, or a plurality of first through holes 51 may be provided and each of them may accommodate separated one optical fiber 34.

A diameter of the optical fiber 34 may change along the axial direction. In the example of Fig. 5, the optical fiber 34 has a small diameter portion where a core 34a is exposed, and a large diameter portion where the core 34a is covered with a clad portion (not illustrated in Fig. 5) and a covering portion 34c. In such a case, the first through hole 51 may have a stepped hole shape as illustrated, that is, the diameter may change along the axial direction depending on a change in the diameter of the optical fiber 34.

The recess 52 is formed so as to open toward a surface (distal end side) facing the layered substrate 37 in the first cap member 50, and accommodates the laser element 32 and the laser driver circuit 33. In the example of Fig. 5, the recess 52 further accommodates the wires 39. The second through hole 53 penetrates the first cap member 50 in the axial direction and accommodates at least a part of the heat dissipation wiring 35.

By providing the first through hole 51, the recess 52, and the second through hole 53 in the first cap member 50, positioning of each accommodation target (optical fiber 34, laser element 32, laser driver circuit 33, heat dissipation wiring 35, and the like) is facilitated, which improves manufacturability. In particular, when the first through hole 51 is configured to have a stepped hole shape as illustrated in Fig. 5, positioning of the optical fiber 34 is further facilitated.

The optical transmitter module 30 is sealed with a molding agent. Fig. 6 illustrates an example of sealing with a molding agent 61.

A second cap member 60 having a tubular shape is attached to the optical transmitter module 30. The second cap member 60 has, for example, a bottomed tubular shape, but may have a tubular shape penetrating without a bottom. The second cap member 60 is made of, for example, resin but may be made of any material. The second cap member 60 is disposed so as to cover, from the distal end side of the optical transmitter module 30, the entire image sensor 31, the layered substrate 37, and the first cap member 50, and the inside of the second cap member 60 is filled with the molding agent 61.

The whole including the second cap member 60 and the molding agent 61 can be disposed inside the hard portion 11a that is not deformed.

Inside the second cap member 60, the image sensor 31, the laser element 32, the laser driver circuit 33, at least a part of the optical fiber 34, at least a part of the heat dissipation wiring 35, at least a part of the electric signal wiring 36, and the layered substrate 37 are disposed. As a result, the image sensor 31, the laser element 32, the laser driver circuit 33, at least a part of the optical fiber 34, at least a part of the heat dissipation wiring 35, at least a part of the electric signal wiring 36, and the layered substrate 37 are fixed to each other with the molding agent 61, and fixing of each component to the layered substrate 37 becomes more reliable. Note that it does not matter if the molding agent 61 is filled into the first cap member 50 (that is, the first through hole 51, the recess 52, and the second through hole 53) or not.

In particular, the molding agent 61 fixes at least a part of the optical fiber 34. As described above, the optical fiber 34 can be prevented from coming off from the laser element 32 by the sealed structure using the molding agent 61.

In this way, the configurations of the optical transmitter module 30 and the endoscope 11 can be further stabilized. In addition, since each component is fixed by filling a relatively small space with the molding agent 61, the entire structure can be more easily downsized.

### Second Embodiment

In the second embodiment, the shape of the first cap member 50 of the first embodiment is changed. Hereinafter, description of parts common to the first embodiment may be omitted.

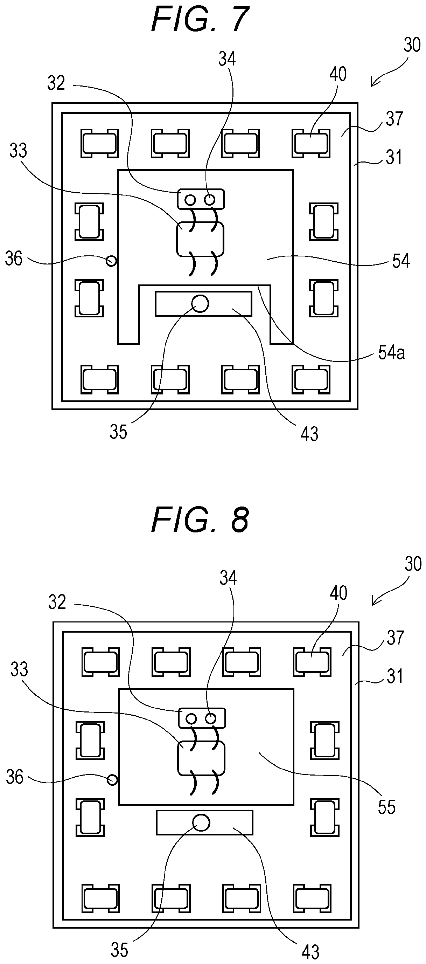

Fig. 7 illustrates a configuration example of an optical transmitter module 30 according to the second embodiment. The optical transmitter module 30 includes a first cap member 54 instead of the first cap member 50 of the first embodiment (Fig. 4). The first cap member 54 has a cutout portion 54a.

The cutout portion 54a covers only a part of the heat dissipation wiring 35 in the circumferential direction. In the example of Fig. 7, the cutout portion 54a covers three directions (upward direction, leftward direction, and rightward direction in the drawing) of the circumferential direction of the heat dissipation wiring 35 and does not cover the remaining one direction (downward direction in the drawing). The cutout portion 54a can also be considered to have a configuration in which a part of the peripheral wall reaches the peripheral wall of the first cap member 50 as a result of the diameter expansion and deformation of the second through hole 53 in the first embodiment (Fig. 5).

According to such a configuration, a process of connecting the heat dissipation wiring 35 to the ground potential pad 43 after attaching the first cap member 54 can be more easily executed, which increases a degree of freedom in the manufacturing process.

### Third Embodiment

In the third embodiment, the shape of the first cap member 50 or the first cap member 54 of the first embodiment or the second embodiment is changed. Hereinafter, description of parts common to the first or second embodiment may be omitted.

Fig. 8 illustrates a configuration example of an optical transmitter module 30 according to the third embodiment. The optical transmitter module 30 includes a first cap member 55 instead of the first cap member 50 of the first embodiment (Fig. 4). The first cap member 55 does not have a configuration corresponding to the second through hole 53 of the first embodiment (Fig. 5), and does not have a cutout such as the cutout portion 54a of the second embodiment (Fig. 7). The shape of the first cap member 55 as viewed from the axial direction (for example, the shape illustrated in Fig. 8) is a convex polygon, or a shape that is convex outward in the entire shape as viewed from the axial direction.

According to such a configuration, a process of connecting the heat dissipation wiring 35 to the ground potential pad 43 after attaching the first cap member 55 can be more easily executed, which increases a degree of freedom in the manufacturing process.

### Fourth Embodiment

In the fourth embodiment, the layered substrate 37 of any one of the first to third embodiments is provided with a cavity. Hereinafter, description of parts common to any one of the first to third embodiment may be omitted.

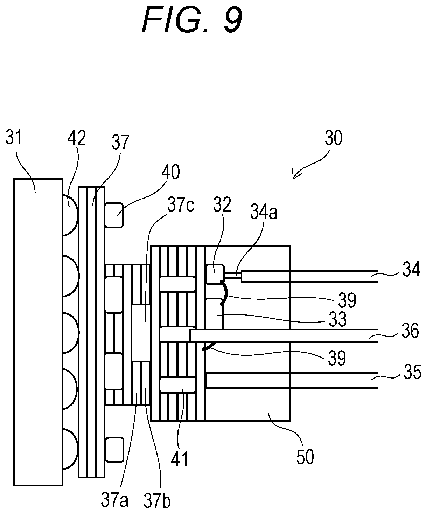

Fig. 9 illustrates a configuration example of an optical transmitter module 30 according to the third embodiment. The layered substrate 37 includes one or more intermediate layers, and in an example of Fig. 9, intermediate layers 37a and 37b are included as parts of the plurality of intermediate layers. Here, the "intermediate layer" means a layer excluding layers located at both ends in the stacking direction among the layers of the substrate constituting the layered substrate 37. In the present embodiment, the stacking direction of the layered substrate 37 is parallel to the axial direction of the endoscope 11.

The intermediate layers 37a and 37b have a cavity 37c. In the example of Fig. 9, the cavity 37c is a cavity penetrating one or more intermediate layers in the stacking direction. This simplifies the manufacturing process, but as a modification, the cavity may be a recess not penetrating the intermediate layer, or may be formed inside the intermediate layer and not opened to the outside.

By forming the cavity 37c in this manner, heat transfer through the layered substrate 37 is suppressed, and for example, an influence of heat generated in the image sensor 31 on the laser element 32 can be reduced. Note that a part of the heat is transferred through the layered substrate 37, but is removed by the heat dissipation wiring 35.

### Other Embodiments

In the embodiments 1 to 4, the first cap members 50, 54, and 55 may be omitted. Even in this case, the optical transmitter module 30 and the endoscope 11 can be more easily downsized with a more stable configuration by fixing with the second cap member 60 and the molding agent 61.

The endoscope system 1 or the endoscope 11 is not limited to the configurations and functions described in the present description and may have configurations and functions of known endoscopes.

The present disclosure includes the following specified matters.

### [Specified matter 1]

An optical transmitter module including:
an image sensor, a laser element, a laser driver circuit, an optical fiber, a heat dissipation wiring, an electric signal wiring, and a layered substrate, wherein
the image sensor, the laser element, the laser driver circuit, at least a part of the optical fiber, at least a part of the heat dissipation wiring, at least a part of the electric signal wiring, and the layered substrate are fixed to each other with a molding agent.

### [Specified matter 2]

The optical transmitter module according to specified matter 1, wherein
the optical transmitter module includes a first cap member made of glass, and
the first cap member includes:
   a first through hole that accommodates at least a part of the optical fiber; and
   a recess that accommodates the laser element and the laser driver circuit.

### [Specified matter 3]

The optical transmitter module according to specified matter 2, wherein
the first cap member further includes a second through hole that accommodates at least a part of the heat dissipation wiring.

### [Specified matter 4]

The optical transmitter module according to specified matter 2, wherein
the first cap member includes a cutout portion that covers only a part of the heat dissipation wiring in the circumferential direction.

### [Specified matter 5]

The optical transmitter module according to specified matter 2, wherein the first cap member does not cover the heat dissipation wiring.

### [Specified matter 6]

The optical transmitter module according to any one of specified matters 2 to 5, wherein
a diameter of the optical fiber changes along an axial direction, and
a diameter of the first through hole changes along an axial direction depending on a change in the diameter of the optical fiber.

### [Specified matter 7]

The optical transmitter module according to any one of specified matters 1 to 6, wherein
the optical transmitter module includes a second cap member having a tubular shape, and
the image sensor, the laser element, the laser driver circuit, at least a part of the optical fiber, at least a part of the heat dissipation wiring, at least a part of the electric signal wiring, and the layered substrate are disposed inside the second cap member, and
the molding agent is filled inside the second cap member.

### [Specified matter 8]

The optical transmitter module according to any one of specified matters 1 to 7, wherein the layered substrate includes an intermediate layer having a cavity.

### [Specified matter 9]

An endoscope including the optical transmitter module according to any one of specified matters 1 to 8.

### Reference Signs List

- 1: Endoscope system
- 10: Endoscope unit
- 11: Endoscope
- 11a: Hard portion
- 11b: Bend portion
- 11c: Flexible portion
- 12: Operation unit
- 13: Connector unit
- 14: Light irradiation means
- 15: Rod
- 20: Processor
- 21: Connector unit
- 30: Optical transmitter module
- 31: Image sensor
- 32: Laser element
- 33: Laser driver circuit
- 34: Optical fiber
- 34a: Core
- 34c: Covering portion
- 35: Heat dissipation wiring
- 36: Electric signal wiring
- 37: Layered substrate
- 37a, 37b: Intermediate layer
- 37c: Cavity
- 39: Wire
- 40: Passive component
- 41: Cable wiring pad
- 43: Ground potential pad
- 50, 54, 55: First cap member
- 54a: Cutout portion
- 51: First through hole
- 52: Recess
- 53: Second through hole
- 60: Second cap member
- 61: Molding agent
All publications, patents, and patent applications cited herein are hereby incorporated by reference in their entirety.

## Claims

1. An optical transmitter module comprising:
an image sensor, a laser element, a laser driver circuit, an optical fiber, a heat dissipation wiring, an electric signal wiring, and a layered substrate, wherein
the image sensor, the laser element, the laser driver circuit, at least a part of the optical fiber, at least a part of the heat dissipation wiring, at least a part of the electric signal wiring, and the layered substrate are fixed to each other with a molding agent.

2. The optical transmitter module according to claim 1, wherein
the optical transmitter module comprises a first cap member made of glass, and
the first cap member includes:
a first through hole that accommodates at least a part of the optical fiber; and
a recess that accommodates the laser element and the laser driver circuit.

3. The optical transmitter module according to claim 2, wherein
the first cap member further includes a second through hole that accommodates at least a part of the heat dissipation wiring.

4. The optical transmitter module according to claim 2, wherein
the first cap member includes a cutout portion that covers only a part of the heat dissipation wiring in the circumferential direction.

5. The optical transmitter module according to claim 2, wherein the first cap member does not cover the heat dissipation wiring.

6. The optical transmitter module according to any one of claims 2 to 5, wherein
a diameter of the optical fiber changes along an axial direction, and
a diameter of the first through hole changes along an axial direction depending on a change in the diameter of the optical fiber.

7. The optical transmitter module according to any one of claims 1 to 6, wherein
the optical transmitter module comprises a second cap member having a tubular shape, and
the image sensor, the laser element, the laser driver circuit, at least a part of the optical fiber, at least a part of the heat dissipation wiring, at least a part of the electric signal wiring, and the layered substrate are disposed inside the second cap member, and
the molding agent is filled inside the second cap member.

8. The optical transmitter module according to any one of claims 1 to 7, wherein the layered substrate includes an intermediate layer having a cavity.

9. An endoscope including the optical transmitter module according to any one of claims 1 to 8.
